# EUROPEAN PATENT APPLICATION

(11) **EP 3 622 975 A1**
(43) Date of publication of application: **18.03.2020**
(21) Application number: 18194298.8
(22) Date of filing: 13.09.2018
(51) Int. Cl.: A61M 1/00, A61F 13/00

(54) **ABSORBENT TUBE AND APPARATUS FOR NEGATIVE PRESSURE WOUND THERAPY**

(71) Applicant: Absorbest AB, 590 39 Kisa (SE)
(72) Inventor: Rovaniemi, Rolf, 590 46 Rimforsa (SE)
(74) Representative: WSL Patentanwälte Partnerschaft mbB

(57) **Abstract**

In order to provide an alternative means for negative pressure wound therapy, which causes less discomfort to a patient, and which is more practical, an absorbent tube for negative pressure wound therapy is provided, which comprises a liquid impermeable sleeve surrounding an absorbent core, the absorbent core comprises a fluid distribution material and a superabsorbent substance, a first connection site for connecting the interior of the liquid impermeable sleeve to a vacuum pump for effecting a negative pressure within the liquid impermeable sleeve, and a second connection site for connecting the interior of the liquid impermeable sleeve to a wound cover so to guide wound exudate into the absorbent core by means of the negative pressure provided by the first connection site, wherein the fluid distribution material is arranged to support a flow of liquid in a from the second connection site to the first connection site. Likewise, an apparatus for negative pressure wound therapy comprising such an absorbent tube is provided.

## Description

The present invention relates to an absorbent tube and an apparatus for negative pressure wound therapy.

Negative pressure wound therapy is used for promoting healing of heavily exuding wounds, burns, and diabetes ulcers. A negative pressure applied to a sealed wound space stimulates perfusion of the wound region, and thus improves nutrition of the wound region and supply of healing friendly body fluids. Furthermore, the negative pressure withdraws wound exudate, including inflammatory agents or harmful substances, from the wound bed.

In order to exert a negative pressure to the wound, the wound is covered by a wound cover. The wound cover is adhesively sealed to the healthy skin surrounding the wound. By connecting the wound cover to a vacuum pump, a negative pressure can be applied to the wound space.

Underneath the wound cover a wound dressing comprising an absorbent core with a superabsorbent material is provided for collecting the wound exudate withdrawn from the wound by the negative pressure. The superabsorbent material has the capability of absorbing a large amount of liquid until being saturated.

However, a regular change of the wound dressing due to the saturation of the absorbent core requires that the wound cover is peeled off from the skin surrounding the wound, which is painful for a patient and might stress the wound thereby hindering wound healing.

Alternatively, in case no absorbent core is provided underneath the wound cover, the wound exudate withdrawn by the negative pressure is collected in a collecting bin, and shall not flow into the vacuum pump so to avoid any damage thereof. Apart from the need for a separator tor separate wound exudate and gas, the collecting bin is often bulky and interferes with a patient's mobility. Furthermore, when in movement the noise of the wound exudate sloshing in the collecting bin causes discomfort to a patient. When using a collecting bin, it has to be regularly purged and disinfected, which means more burden to a patient.

When compared to the prior art it is an object of the present invention to provide an alternative means for negative pressure wound therapy, which causes less discomfort to a patient, and which is more practical.

At least one of the above objects is solved by an absorbent tube for negative pressure wound therapy comprising a liquid impermeable sleeve surrounding an absorbent core, the absorbent core comprises a fluid distribution material and a superabsorbent substance, a first connection site for connecting the interior of the liquid impermeable sleeve to a vacuum pump for effecting a negative pressure within the liquid impermeable sleeve, and a second connection site for connecting the interior of the liquid impermeable sleeve to a wound cover so to guide wound exudate into the absorbent core by means of the negative pressure provided by the first connection site, wherein the fluid distribution material is arranged to support a flow of liquid in a from the second connection site to the first connection site.

The liquid impermeable sleeve is a barrier, which provides an encapsulated volume encompassing the absorbent core. The liquid impermeable sleeve provides sufficient space not to hinder an increase in volume of the absorbent core due to the absorption of wound exudate. Preferably the absorbent core is loosely arranged within the sleeve. Alternatively, the absorbent core is affixed to the liquid impermeable sleeve, e.g. by an adhesive. The liquid impermeable sleeve protects the absorbent core, protects the environment, and protects the clothing of a patient using the absorbent tube.

The liquid impermeable sleeve can be formed by folding a sheet of a fluid impermeable material around the absorbent core and by joining the folded sections so to form a fluid impermeable seam. Alternatively, the liquid impermeable sleeve can be formed of two sheets of a fluid impermeable material. One sheet of the fluid impermeable material is laid on top of the absorbent core and one sheet of the fluid impermeable material is laid underneath the absorbent core. The overlapping edges of the sleeve formed by the upper and lower sheets of fluid impermeable material are joint together so to form a circumferential fluid impermeable seam.

Further alternatively, the liquid impermeable sleeve can be formed by folding two sheets of a fluid impermeable material. One sheet of fluid impermeable material is arranged underneath the absorbent core. The sheet covers the entire bottom surface of the absorbent core. At least two edges of the sheet are folded around the absorbent core, wherein the at least two folding sections of the sheet are arranged on top of the absorbent core. The second sheet of fluid impermeable material is arranged on top of the absorbent core and underneath or on top of the at least two folded sections. The at least two folded sections are joint together with the second sheet of fluid impermeable material so to form a fluid impermeable seam. The seam can be formed by hot melting or welding. In a further alternative embodiment, the liquid impermeable sleeve can be formed by a hose made of a fluid impermeable material, wherein, after insertion of the absorbent core, the ends of the hose are sealed by a seam so to form a closed pouch.

During use, the liquid impermeable sleeve functions as a container for the absorbent core, but also provides a fluid communication between the vacuum pump and the sealed wound cover. The first connection site is connected to the vacuum pump, and the second connection site is connected to the wound cover. Accordingly, a negative pressure exerted by the vacuum pump via the first connection site to the absorbent tube is also exerted to the wound area sealed by the wound cover via the second connection site. Wound exudate is thus guided via the second connection site into the absorbent tube by means of the negative pressure applied via the first connection site. Wound exudate originating from a sealed wound and guided into the absorbent tube by means of the negative pressure is absorbed by the superabsorbent substance of the absorbent core. The bonded wound exudate causes less noise when moving the absorbent tube.

However, in order to support the absorbent core to take advantage of its full absorbing capacity and to hinder that the flow of fluid is hindered by swollen superabsorbent substance, a phenomenon which is sometimes called gel-blocking, the fluid distribution substance is provided. In the meaning of the present invention the fluid distribution substance is configured to support a flow of liquid in a direction from the second connection site to the first connection site. By being guided by means of the fluid distribution substance the wound exudate can thus get more uniformly in contact with the superabsorbent substance, and thus can be absorbed more evenly. Preferably, the fluid distribution substance provides a flow resistance, which is lower than the flow resistance of the superabsorbent substance. Further preferably, the fluid distribution substance comprises a capillary attraction, which is larger than the capillary attraction of the superabsorbent substance.

In the sense of the present invention a guidance of flow of wound exudate in a direction from the second connection site towards the first connection site means that, in use of the absorbent tube, the flow of liquid in a direction from the second connection site towards the first connection site is faster than in a direction, which is perpendicular thereto.

Once the absorbent core is saturated the entire absorbent tube or the absorbent core can be replaced without the need of peeling off the wound cover from a patient's skin, thereby causing less discomfort and pain to a patient. Furthermore, since the replacement of the absorbent tube or the absorbent core, respectively, does not require any medical education, there is no need for involving any medical staff. By being more independent from medical assistance, a patient gains more freedom.

In an embodiment according to the present invention the absorbent core has an elongated shape, wherein the fluid distribution material is arranged to support a flow of liquid in the longitudinal direction (L) of the absorbent core. Due to its elongated shape the length of the absorbent core is larger than its width and height. Preferably, the liquid impermeable sleeve surrounding the absorbent core has an elongated shape, too.

Preferably, in a longitudinal view, the first connection site and the second connection site are arranged on opposing ends of the elongated absorbent core. The longitudinal direction of the absorbent core corresponds to or is parallel to the direction from the second connection site towards the first connection site.

According to an embodiment of the present invention the liquid impermeable sleeve consists of a flexible film, preferably made from polyurethane or polyolefin, wherein preferably the liquid impermeable sleeve has a material thickness in the range from 15 µm to 150 µm. By being flexible the liquid impermeable sleeve is less prone to breakage and at the same time does not hinder an increase in volume of the absorbent core due to the absorption of wound exudate by the superabsorbent substance. Furthermore, a flexible sleeve allows an adaption of the form of the sleeve and is less bulky than a sleeve formed as a housing.

However, the liquid permeable sleeve can by formed as a housing, e.g. formed by injection molding, if needed, or can be inserted into an additional housing, if a more robust protection is required.

Preferably, the material of the liquid impermeable sleeve is elastic. For instance, the liquid impermeable sleeve can be elastically stretched about 50 % to 700 %, preferably the liquid impermeable sleeve can be elastically stretched about 200 % to 600 %.

In a further embodiment of the present invention, alternatively or supplementary, the material of the liquid impermeable sleeve has a moisture vapor transmission rate in a range from 200 g/(m² * 24h) to 18000 g/(m² * 24 h), preferably in a range from 500 g/(m² * 24h) to 4000 g/(m² * 24h), further preferably in a range from 1000 g/(m² * 24h) to 3000 g/(m² * 24h). The moisture vapor transmission rate (MVTR) is a measure of the water vapor permeability of the liquid impermeable sleeve. Upon absorption the weight of the absorbent core increases by the amount of absorbed wound exudate. A MVTR in a range from 200 g/(m² * 24h) to 18000 g/(m² * 24h) helps to reduce the weight of the absorbent core during operation by allowing water vapor to migrate out of the liquid impermeable sleeve. Less weight means more comfort to a patient. Furthermore, less weight of the absorbent core means less burden to the liquid impermeable sleeve, and, thus, helps to increase the durability of the absorbent tube. In an embodiment the moisture vapor transmission rate is measured by applying the standard test method ASTM E96.

In an embodiment the fluid distribution material in the sense of the present application consists of or comprises one or more materials selected from a group consisting of: 1.) fluff, 2.) defibrated cellulose, as bleached or unbleached chemical pulp or thermomechanical pulp, 3.) tissue paper, 4.) spunlaced cellulose fibers, or spunlaced viscose fibers or spunlaced synthetic fibers, or spunlaced mixture of cellulose fiber and/or viscose fibers and/or synthetic fibers, 5.) textile fabric, 6.) needle punched nonwoven or needle punched textile, 7.) spunbond nonwoven viscose, 8.) chemically bonded nonwoven, 9.) air-laid material based on fluff mainly bonded with steam and/or water to a mix of synthetic fibers, 10.) air-laid material with a binder or a mixture of cellulose and synthetic fibers, 11.) dry-web material, and 12.) spunlaced or needle-punched non-woven consisting of 33 % by weight polyester and 67 % by weight viscose. Preferably, the spunlaced or needle-punched non-woven consisting of 33 % by weight polyester and 67 % by weight viscose is material offered under the trademark "Fibrella" having a weight in a range from 30 gsm to 150 gsm. Preferably, a layer of Fibrella has a material thickness in the range from 0.44 mm to 0.70 mm. Dry-web is an air-bonded non-woven having a weight in a range from 25 gsm to 55 gsm. Preferably, dry-web has a tensile strength in a range from 8 N/5cm to 47 N/5cm, preferably a tensile strength in a range from 17 N/5cm to 42 N/5cm measured in accordance with standard test method according to ISO 9073-3. Further, preferably dry-web has a liquid strike-through time for simulated urine of maximum 2.5 seconds measured in accordance with standard test method WSP 70.3 provided by EDANA (European Disposables And Nonwovens Association)/ INDA (International Nonwovens And Disposables Association), and/or a wetback amount of liquid maximum 0.2 g measured in accordance with standard test method WSP 80.10 provided by EDANA/INDA.

Whenever in the context of the present application a weight is given in grammes per square meter (g/m²) it means that a square meter of the respective material weights to the amount of grammes given. This weight in the paper industry is commonly denoted as paper weight, wherein the unit grammes per square meter is identical to gsm. It is commonly named an area weight. Whenever a weight is given in the context of this application it is given for the material in its unwetted state.

Superabsorbent substances in the sense of the present application are materials that have the ability to absorb and retain large volumes of water and aqueous solutions. Superabsorbent substances falling into this category are for example modified starch, polymer like polyvinyl alcohol (PVA), polyethylene oxide (PEO) which are all hydrophilic and have a high affinity for water. When lightly chemically or physically cross-linked, these polymers are water-swellable but not water-soluble.

In an embodiment the superabsorbent substance in the sense of the present application is a superabsorbent polymer made from partially neutralised, lightly cross-linked polyacrylic acid, which has been proven to give the best performance vs. cost ratio. Those superabsorbent polymers in an embodiment are manufactured at low solids levels for quality economic reasons and are dried and milled into granular white solids. In water they swell to a rubbery gel. Superabsorbent polymers may absorb up to 500 times their weight of water. The superabsorbent substance can be provided in form of fibres, particles, granulates or gel.

The superabsorbent polymers can have an outer shell which promotes fluid distribution.

According to a further embodiment of the present application the superabsorbent substance consists of or comprises one or more materials selected from a group consisting of: 1.) sodium polyacrylate crosslinked polymer, 2.) modified starch, 3.) polymer-like polyvinyl alcohol, and 4.) polyethylene oxide, preferably the superabsorbent substance is provided in form of fibers, particles, granulates or gel.

In an embodiment of the present invention the fluid distribution material and the superabsorbent substance form a mixture. The contents of the fluid distribution material enables a liquid transport in a direction from the second connection site towards the first connection site, and avoids a blocking of liquid transport in the longitudinal direction of the absorbent core. For instance, in the un-wetted state of the absorbent core, the fluid distribution material amounts 10 % to 40 % by weight of the absorbent core, and the superabsorbent substance amounts 90 % to 60 % by weight of the absorbent core. Alternative or supplementary to the ratio of the amounts of fluid distribution material and superabsorbent substance contained in the absorbent core, a flow of liquid in a direction from the second connection site towards the first connection site can be achieved by providing the fluid distribution material in form of fibers mixed with superabsorbent polymers. Preferably, when mixed with superabsorbent polymers more than 50 % of the fibers of the fluid distribution substance have a length in one direction of 0.5 mm to 2 mm.

The mixture of superabsorbent substance and fluid distribution material can be surrounded by a fluid permeable sleeve forming a boundary of the absorbent core.

According to a further embodiment of the present invention the fluid distribution material forms at least one fluid distribution layer and the superabsorbent substance forms part of at least one absorbent layer, wherein the at least one fluid distribution layer and the at least one absorbent layer are stacked on top of each other, wherein the at least one fluid distribution layer is arranged to support a flow of liquid primarily in a direction parallel to the boundary between the at least one absorbent layer and the at least one distribution layer. The fluid distribution layer forms a pathway for the wound exudate to flow in a direction parallel to the boundary between two adjacent layers of the absorbent core, thereby allowing the wound exudate to distribute over the length of the absorbent core and to be absorbed more uniformly over the longitudinal extension of the absorbent core. By forming separated layers the swollen superabsorbent substance does not block the pathway provided by the fluid distribution layer, and gel blocking becomes less relevant. Preferably, in a direction parallel to the boundary between two adjacent layers of the absorbent core, the at least one fluid distribution layer has a flow resistance, which is lower than the flow resistance of the at least one absorbent layer.

Preferably, the at least one fluid distribution layer has weight in a range from 50 gsm to 200 gsm and/or the at least one absorbent layer has a weight in a range from 100 gsm to 400 gsm.

In an embodiment of the present invention, alternatively or supplementary, multiple fluid distribution layers and multiple absorbent layers are alternately stacked on top of each other, wherein preferably the number of absorbent layers equals the number of the fluid distribution layers or the number of absorbent layers exceeds the number of fluid distribution layers by one or the number of absorbent layers is by one smaller than the number of fluid distribution layers. Accordingly, the stack of fluid distribution layers and absorbent layers can be formed by multiple fluid distribution layers each provided between two adjacent absorbent layers. Alternatively, the stack can be formed by multiple absorbent layers each provided by between two adjacent fluid distribution layers. Further alternatively, the two outermost layers of the stack can be made from different types of layers.

Preferably, the number of absorbent layers can vary from 1 to 6.

In an embodiment of the present invention, alternatively or supplementary, a carrier layer is arranged between the at least one fluid distribution layer and the at least one absorbent layer. The carrier layer supports the structural integrity of the absorbent core. Preferably, a carrier layer is arranged between each pair of a fluid distribution layer and an absorbent layer.

According to an embodiment of the present invention, alternatively or supplementary, the carrier layer consists of or comprises one or more materials selected from a group consisting of: 1.) tissue paper, 2.) spunlaced cellulose fibers, or spunlaced viscose fibers or spunlaced synthetic fibers, or spunlaced mixture of cellulose fibers and/or viscose fibers and/or synthetic fibers, 3.) textile fabric, 4.) needle punched nonwoven or needle punched textile, 5.) spun-bond based on a nonwoven material of mainly viscose, 6.) chemically bonded nonwoven, 7.) air-laid material based on fluff mainly bonded with steam and/or water to a mix of synthetic fibers, and 8.) air-laid material with a binder or a mixture of cellulose and synthetic fibers.

In an embodiment of an absorbent tube according to the present invention, the carrier layer is a hydrophilic or hydrophobic non-woven material, preferably the hydrophilic or hydrophobic non-woven material is one of a polypropylene (PP) spunbond, a combined non-woven fabric comprising a layer of a meltblown between two layers of a spunbond non-woven (SMS), a hydroentangled non-woven or a combined non-woven fabric comprising, in alternating order, three spunbond non-woven layers and two meltblown non-woven layers (SMSMS).

In a further embodiment of an absorbent tube according to the present invention, the carrier layer is a non-woven material, a perforated sheet, a perforated sheet laminated on a non-woven material, a fine net or screen, or a combination thereof. In an embodiment the non-woven material of the carrier layer contains polyethylene (PE), polyethylene terephthalate (PET), polypropylene (PP), polyamide or polytetrafluoroethylene (PTFE). In an embodiment the perforated sheet contains polyethylene (PE), polyethylene terephthalate (PET), polypropylene (PP), poly-amide or polytetrafluoroethylene (PTFE). In an embodiment the perforated sheet laminated on a non-woven material contains polyethylene (PE), polyethylene terephthalate (PET), polypropylene (PP), polyamide or polytetrafluoroethylene (PTFE). In an embodiment the fine net or screen contains polyethylene (PE), polyethylene terephthalate (PET), polypropylene (PP), polyamide or polytetrafluoroethylene (PTFE).

In the meaning of the present invention a non-woven is a material made of at least one layer of undirected fibers that have been formed to a web and consolidated in a next step. In particular, consolidation of the non-woven material may be achieved by friction and/or cohesion and/or adhesion, for example by needling, felting, spunlacing, melting or heat embossing. When spunlacing is achieved by using water jets the non-woven is called a hydroentangled non-woven.

If compared to tissue paper a material will be considered a non-woven fabric in the sense of the present application once more than 50 % of the mass of its fiber components consist of fibers having a ratio of their length to their diameter of more than 300. Alternatively, the material will be considered a non-woven fabric in the sense of the present application if this condition is not fulfilled, but if more than 30 % of the mass of its fiber components consist of fibers having a ratio of their length to their diameter of more than 300 and its density is lower than 0.4 g / cm³. This is deemed to be in conformity with EN 29 092.

In an embodiment of the present invention, alternatively or supplementary, the carrier layer is impermeable for the superabsorbent substance, in its un-wetted state. Thus, the carrier layers form a barrier hindering the superabsorbent substance, in its un-wetted state, to migrate into an adjacent layer or migrate out of the absorbent layer.

According to a further embodiment of the present invention, alternatively or supplementary, at least one of the lowermost and uppermost surfaces of the stack formed by the at least one fluid distribution layer or the at least one absorbent layer is formed by a carrier layer. Preferably the two opposing outermost surfaces of the stack formed by the at least one fluid distribution layer or the at least one absorbent layer are formed by a carrier layer. The carrier layers form a boundary of the absorbent core, and thus support the structural integrity of the absorbent core.

In an embodiment of the present invention, alternatively or supplementary, the fluid distribution layer comprises a density which is smaller than the density of the carrier layer and/or, in a direction perpendicular to the longitudinal direction of the absorbent core, the fluid distribution layer comprises an extension which is larger than the extension of the carrier layer, and/or the fluid distribution layer comprises a larger amount of synthetic fibers than the carrier layer.

For connecting the absorbent tube to a vacuum pump and to a sealed wound cover, the absorbent tube comprises a first connection site and a second connection site. However, for effectively providing a flow of liquid by means of the negative pressure, according to an embodiment of the present invention the first connection site for connecting the interior of the liquid impermeable sleeve to a vacuum pump for effecting a negative pressure within the liquid impermeable sleeve comprises a tubular member, a first end of which rests in the outermost layer or on the outermost surface of the stack formed by the at least one fluid distribution layer and the at least one absorbent layer. When resting in the outermost layer or on the outermost surface of the stack a flow of liquid is promoted through the absorbent core. Preferably, the outermost surface of the stack is formed by a carrier layer, which hinders that parts of the absorbent core or particles, in particular superabsorbent particles, fibers, or the like, are sucked into the first end of the tubular member, which can block the fluid communication with the vacuum pump or may damage the vacuum pump. Further preferably, the layer underneath the outermost carrier layer is an absorbent layer.

In a further embodiment, alternatively or supplementary, the second connection site for connecting the interior of the liquid impermeable sleeve to a wound cover so to guide wound exudate into the absorbent core by means of the negative pressure provided by the first connection site comprises a tubular member, a first end of which rests in the innermost layer or on the outer surface of the innermost layer of the stack formed by the at least one fluid distribution layer and the at least one absorbent layer. The tubular member resting in the innermost layer or on top of the innermost layer of the stack, the wound exudate guided into the absorbent core is directly guided into the absorbent core. Preferably, the innermost layer is a fluid distribution layer, such that upon entrance the wound exudate guided into the absorbent core can be further distributed in the longitudinal direction of the absorbent core.

Preferably, the first end of the tubular member of the first connection site and the first end of the tubular member of the second connection site are arranged to the outermost layers of the absorbent core in a diametrical manner. For instance, the first end of the tubular member of the first connection site can be arranged on top of the outer surface of the uppermost layer or in the uppermost layer of the absorbent core, while the first end of the tubular member of the second connection site is arranged on top of the outer surface of the lowermost layer or in the lowermost layer of the absorbent core.

Alternatively or supplementary, the tubular member of the first connection site and/or the tubular member of the second connection site may comprise a sieve or net. The sieve or net hinders particles to enter the respective tubular member.

Preferably the first connection site comprises a one-way valve allowing a flow of liquid in the direction outwardly from the absorbent tube towards the vacuum pump. Alternatively or supplementary, the second connection site can comprise a one-way valve allowing a flow of liquid in the direction from the sealed wound cover into the absorbent tube. Likewise, a one-way valve can be provided with a vacuum pump, a wound cover or within the connection between a wound cover and the second connection site, or within the connection between a vacuum pump and the first connection site. According to a further embodiment of the present invention, each of the first connection site and the second connection site comprises a one-way valve.

The present invention is further directed to an apparatus for negative pressure wound therapy comprising a vacuum pump, a wound cover applicable to a wound in a human or animal skin, and an absorbent tube according to the present invention connecting the vacuum pump to the wound cover. The wound cover can be sealed to a patient's skin surrounding a wound, preferably the wound cover can be gas-tightly sealed to a patient's skin surrounding a wound. The wound cover can be transparent so to allow a visual inspection of the wound without the need of peeling the wound cover off a patient's skin.

In order to improve control of the negative pressure applied to the wound cover, in an embodiment of the present invention a pressure sensor is provided in the connection between the second connection site and the wound cover, wherein the apparatus further comprises a control unit for controlling the vacuum pump, the control unit is arranged to receive a signal from the pressure signal and to control the vacuum pump by considering the pressure measured by the pressure sensor.

In a further embodiment of the present invention, alternatively or supplementary, in use, a filler material is arranged between the wound cover and a patient's wound. The filler material helps to apply a negative pressure provided via the second connection site more uniformly over the wound area. Optionally, the filler material can comprise an antibacterial substance. The antibacterial substance helps to suppress inflammatory agents. The filler material can comprise or consist of one or more materials selected from a group consisting of collagen, foam, cellulose, and synthetic fibers.

Further advantages, features and applications of the present disclosure will become apparent from the following description of embodiments and the corresponding figures attached. The foregoing as well as the following detailed description of the embodiments will be better understood when read in conjunction with the appended drawings. It should be understood that the embodiments depicted are not limited to the precise arrangements and instrumentalities shown.
- Figure 1: schematically shows an apparatus for negative wound pressure therapy comprising an absorbent tube according to a first embodiment of the present invention,
- Figure 2: schematically shows a longitudinal sectional view of an absorbent tube according to a further embodiment of the present invention,
- Figure 3: schematically shows a longitudinal sectional view of an absorbent tube according to a further embodiment of the present invention, and
- Figure 4: schematically shows a longitudinal sectional view of an absorbent tube according to a further embodiment of the present invention.

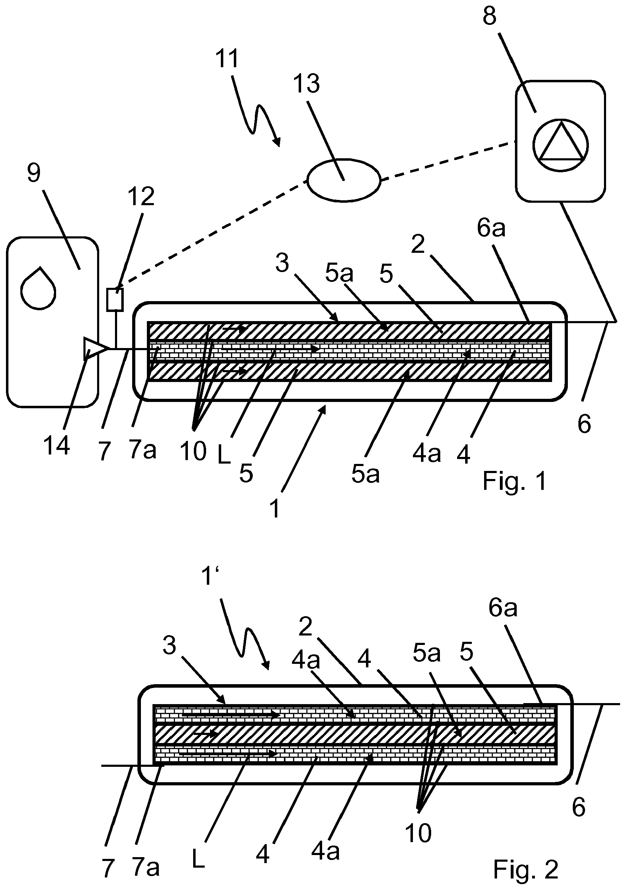

In terms of the present description like reference numerals refer to like elements. Furthermore, for the purpose of original disclosure, the absorbent tube 1 of figure 1 is replaceable by each of the absorbent tubes 1', 1", 1'" shown in figures 2 to 4.

Figure 1 schematically shows an apparatus 11 for negative pressure wound therapy according to an embodiment of the present invention. The apparatus 11 comprises an absorbent tube 1, a wound cover 9, and a vacuum pump 8. The absorbent tube 1 connects the wound cover 9 to the vacuum pump 8 so to allow an evacuation of a wound space covered by the wound cover 9 by means of a negative pressure effected by the vacuum pump 8.

The absorbent tube 1 comprises a liquid impermeable sleeve 2 surrounding a longitudinal absorbent core 3. The absorbent core 3, the liquid impermeable sleeve 2, and, thus, the absorbent tube 1, each have an elongated shape. The longitudinal direction of the elongated absorbent core 3 is indicated by an arrow and referenced by the character "L".

The liquid impermeable sleeve 2 protects the absorbent core 3 and is made of a flexible, fluid impermeable material. Accordingly, due to its flexibility the liquid impermeable sleeve 2 can be adapted to a shape of a part of a patient's body to which the absorbent tube 1 should be attached to for wearing. Furthermore, the elasticity of the liquid impermeable sleeve 2 does not hinder an increase in volume of the absorbent core 3 when absorbing wound exudate.

The interior of the liquid impermeable sleeve 2 is connected to the vacuum pump 8 via a first connection site 6. Likewise, the interior of the liquid impermeable sleeve 2 is connected to the wound cover 9 via a second connection site 7. Accordingly, a negative pressure generated by the vacuum pump 8 is applied to the interior of the liquid impermeable sleeve 2 via the first connection site 6 and is further applied to a wound space sealed by the wound cover 9 via the second connection site 7. Wound exudate released from the wound is withdrawn by the negative pressure through the second connection site 7 into the liquid impermeable sleeve 2, where it is absorbed by the absorbent core 3.

The absorbent core 3 comprises a fluid distribution material 4 and a superabsorbent substance 5.The fluid distribution material 4 forms a fluid distribution layer 4a, which is arranged between two absorbent layers 5a. The absorbent layers 5a are partially formed by the superabsorbent substance. While the superabsorbent substance 5 has the ability to absorb large amounts of wound exudate, the fluid distribution material 4 supports a flow of wound exudate in a direction from the second connection site 7 towards the first connection, which in the present embodiment corresponds to the longitudinal direction of the absorbent core 3.

Between each of fluid distribution layers 4a and the absorbent layers 5a there is a carrier layer 10 provided, which supports the structural integrity of the absorbent core 3. On top of the opposing outermost surfaces of the absorbent layers 5a there is respectively provided a carrier layer 10, too. The carrier layers 10 hinder particles to migrate into adjacent layers 4a, or out of the absorbent core 3. Furthermore, the carrier layers 10 forming the opposing outermost surfaces of the stack of layers also hinder that parts of the absorbent core 3 are sucked into a first end of a tubular member 6a of the first connection site 6, which rests on top of the outermost layer of the stack formed by the absorbent layers 5a and the fluid distribution layer 4a.

By being arranged on top of the outermost surface of one of the absorbent layers 5a, a negative pressure provided via the first connection site 6 is directly applied to the absorbent layer 5a thereby supporting a flow of fluid through the absorbent core 3. At the opposing end of the absorbent core 3 the second connecting site 7 also comprises a tubular member 7a with a first member, which, however, rests in the in the innermost layer of the stack forming the absorbent core 3. The innermost layer of this embodiment is the fluid distribution layer 4a, such that wound exudate withdrawn from a wound is directly guided into the fluid distribution layer 4a thereby promoting a distribution of the fluid via the distribution layer 4a of the absorbent core 3, and in a direction from the second connection site 7 towards the first connection site 6.

In order to hinder a flow of wound exudate outward of the absorbent tube and back into the sealed wound space, the connection between the wound cover 9 and the absorbent tube 1 comprises a one-way valve 14 permitting only a flow of fluid in a direction from the wound cove towards the absorbent tube 1.

The wound cover 9 is transparent to enable a visual inspection of the wound without the need of peeling the wound cover 9 off a patient's skin.

In order to control the negative pressure actually exerted on a sealed wound space, the apparatus 11 further comprises a pressure sensor 12 and a control unit 13. The pressure sensor 12 is arranged in the connection between the wound cover 9 and the absorbent tube 1. The pressure 12 is arranged to measure a pressure within the connection line. The control unit 13 is configured to control the vacuum pump 8 by considering the pressure determined by the pressure sensor 12.

Figure 2 schematically shows a longitudinal section of an absorbent tube 1' according to an alternative embodiment of the present invention. The absorbent tube 1' of figure 2 differs from the absorbent tube 1' of figure 1 in that the fluid distribution material forms two fluid distribution layers 4a, and the superabsorbent substance 5 forms part of one absorbent layer 5a. The absorbent layer 5a is arranged between the two fluid distribution layers 4a thereby forming a stack of absorbent and fluid distribution layers 4a, 5a. The first end of the tubular member 7a of the second connection site 7 is arranged on top of the surface of the lowermost fluid distribution layer 4a, while the first end of the tubular member 6a of the first connection site 6 is arranged on top of the surface of the uppermost fluid distribution layer 4a. The first end of tubular member 6a of the first connection site 6 and the first end of the tubular member 7a of the second connection site 7 are arranged diametrically to each other.

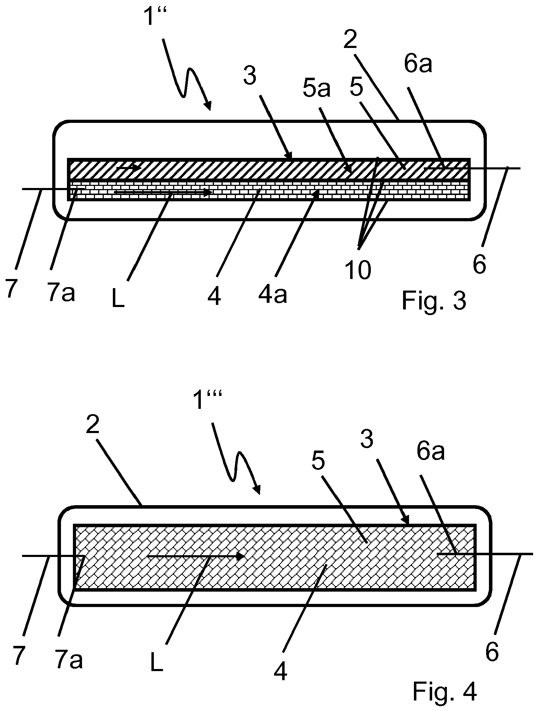

Figure 3 schematically shows a longitudinal section of an absorbent tube 1" according to yet another embodiment of the present invention. The absorbent tube 1" of figure 3 differs from the absorbent tube 1' of figure 2 by one fluid distribution layer 4a formed by the fluid distribution material 4 and one absorbent layer 5a partly formed by the superabsorbent substance 5. The fluid distribution layer 4a and the absorbent layer 5a are stacked on top of each other. Further different to the embodiment of figure 2, the first end of the tubular member 6a of the first connection site 6 for connecting the liquid impermeable sleeve 2 to a vacuum pump 8 rests in the absorbent layer 5a. Likewise, the first end of the tubular member 7a of the second connection site 7 for connecting the liquid impermeable sleeve 2 to a wound cover 9 is arranged within in the fluid distribution layer 4a. Both, the end of the tubular member 6a of the first connection site 6 and the end of the tubular member 7a of the second connection site are provided on opposing ends of the absorbent core 3.

For purposes of original disclosure, it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations of features is only omitted in order to provide readability of the description.

While the disclosure has been described with respect to a limited number of embodiments, it will be understood that the disclosure of which is not limited to those embodiments. Other embodiments comprising various changes do not depart from the scope of the disclosure. In particular, the description of preferred embodiments shall not be understood to be limited to what is explicitly shown and described in the specification and drawings but shall encompasses the disclosure of the specification and drawings as a whole.

List of reference numerals
- 1, 1', 1", 1'": absorbent tube
- 2: liquid impermeable sleeve
- 3: absorbent core
- 4: fluid distribution material
- 4a: fluid distribution layer
- 5: superabsorbent substance
- 5a: absorbent layer
- 6: first connection site
- 6a: tubular member
- 7: second connection site
- 7a: tubular member
- 8: vacuum pump
- 9: sealed wound cover
- 10: carrier layer
- 11: apparatus for negative pressure wound therapy
- 12: pressure sensor
- 13: control unit for controlling the vacuum pump 8
- 14: one-way valve
- L: longitudinal direction of absorbent core 3

## Claims

1. An absorbent tube (1, 1', 1", 1"') for negative pressure wound therapy comprising a liquid impermeable sleeve (2) surrounding an absorbent core (3), wherein
the absorbent core (3) comprises a fluid distribution material (4) and a superabsorbent substance (5),
a first connection site (6) for connecting the interior of the liquid impermeable sleeve (2) to a vacuum pump (8) for effecting a negative pressure within the liquid impermeable sleeve (2), and
a second connection site (7) for connecting the interior of the liquid impermeable sleeve (2) to a wound cover (9) so to guide wound exudate into the absorbent core (3) by means of the negative pressure provided at the first connection site (6), wherein
the fluid distribution material (4) is arranged to support a flow of liquid in a direction from the second connection site (7) to the first connection site (6).

2. The absorbent tube (1, 1', 1", 1"') according to claim 1, **characterized in that** the absorbent core (3) has an elongated shape, wherein the fluid distribution material is arranged to support a flow of liquid in a longitudinal direction (L) of the absorbent core (3).

3. The absorbent tube (1, 1', 1", 1"') according to claim 1 or 2, **characterized in that** the liquid impermeable sleeve (2) consists of a flexible film (2a), preferably made from polyurethane or polyolefin, wherein the liquid impermeable sleeve (2) preferably has a material thickness in the range from 15 µm to 150 µm, further preferably the material of the liquid impermeable sleeve (2) has a moisture vapor transmission rate in a range from 200 g/(m² * 24h) to 18000 g/(m² * 24 h), preferably in a range from 500 g/(m² * 24h) to 4000 g/(m² * 24h), further preferably in a range from 1000 g/(m² * 24h) to 3000 g/(m² * 24h).

4. The absorbent tube (1, 1', 1", 1"') according to one of claims 1 to 3, **characterized in that** the fluid distribution material (4) consists of or comprises one or more materials selected from a group consisting of: 1.) fluff, 2.) defibrated cellulose as bleached or unbleached chemical pulp, or thermomechanical pulp, 3.) tissue paper, 4.) spunlaced cellulose fibers, or spunlaced viscose fibers or spunlaced synthetic fibers, or spunlaced mixture of cellulose fibers and/or viscose fibers and/or synthetic fibers, 5.) textile fabric, 6.) needle punched nonwoven or needle punched textile, 7.) spunbond nonwoven material of mainly viscose, 8.) chemically bonded nonwoven, 9.) air-laid material based on fluff mainly bonded with steam and/or water to a mix of synthetic fibers, 10.) air-laid material with a binder or a mixture of cellulose and synthetic fibers, 11.) a dry-web material, and 12.) spunlaced or needle-punched non-woven consisting of 33 % by weight polyester and 67 % by weight viscose.

5. The absorbent tube (1"') according to one of claims 1 to 4, **characterized in that** the fluid distribution material (4) and the superabsorbent substance (5) form a mixture.

6. The absorbent tube (1, 1', 1") according to one of claims 1 to 4, **characterized in that** the fluid distribution material (4) forms at least one fluid distribution layer (4a) and the superabsorbent substance (5) forms part of at least one absorbent layer (5a), wherein the at least one fluid distribution layer (4a) and the at least one absorbent layer (5a) are stacked on top of each other, wherein the at least one fluid distribution layer (4a) is configured to support a flow of liquid primarily in a direction parallel to the boundary between the at least one absorbent layer (5a) and the fluid distribution layer (4a).

7. The absorbent tube (1, 1', 1") according to claim 6, **characterized in that** a carrier layer (10) is arranged between the at least one fluid distribution layer (4a) and the at least one absorbent layer (5a).

8. The absorbent tube (1, 1', 1") according to claim 6 or 7, **characterized in that** multiple fluid distribution layers (4a) and multiple absorbent layers (5a) are alternately stacked on top of each other, wherein preferably the number of absorbent layers (5a) equals the number of the fluid distribution layers (4a) or the number of absorbent layers (5a) exceeds the number of fluid distribution layers (4a) by one or the number of absorbent layers (5a) is by one smaller than the number of fluid distribution layers (4a), wherein preferably the number of absorbent layers (5a) is in a range from 1 to 6.

9. The absorbent tube (1, 1', 1") according to one of claims 6 to 8, **characterized in that** at least one of the lowermost and uppermost surfaces of the stack formed by the at least one fluid distribution layer (4a) and the at least one absorbent layer (5a) is formed by a carrier layer (10).

10. The absorbent tube (1, 1', 1") according to one of claims 6 to 9, **characterized in that** the carrier layer (10) consists of or comprises one or more materials selected from a group consisting of: 1.) tissue paper, 2.) spunlaced cellulose fibers, or spunlaced viscose fibers, or spunlaced synthetic fibers, or spunlaced mixture of cellulose fibers and/or viscose fibers and/or synthetic fibers, 3.) textile fabric, 4.) needle punched nonwoven or needle punched textile, 5.) spunbond nonwoven material of mainly viscose, 6.) chemically bonded nonwoven, 7.) air-laid material based on fluff mainly bonded with steam and/or water to a mix of synthetic fibers, and 8.) air-laid material based on cellulose with a binder or a mixture of cellulose and synthetic fibers.

11. The absorbent tube (1, 1', 1") according to one of claims 6 to 10, **characterized in that** the carrier layer (10) is impermeable for the superabsorbent substance (5), in its un-wetted state.

12. The absorbent tube (1, 1', 1") according to one of claims 7 to 11, **characterized in that** the fluid distribution layer (4a) comprises a density which is smaller than the density of the carrier layer (10) and/or, in a direction perpendicular to the longitudinal direction (L) of the absorbent core (3), the fluid distribution layer (4a) comprises an extension which is larger than the extension of the carrier layer (10), and/or the fluid distribution layer (4a) comprises a larger amount of synthetic fibers than the carrier layer (10).

13. The absorbent tube (1, 1', 1") according to one of claims 6 to 12, **characterized in that** the first connection site (6) for connecting the interior of the liquid impermeable sleeve (2) to a vacuum pump (8) for effecting a negative pressure within the liquid impermeable sleeve (2) comprises a tubular member (6a), a first end of which rests in the outermost layer or on the outermost surface of the stack formed by the at least one fluid distribution layer (4a) and the at least one absorbent layer (5a), and/or the second connection site (7) for connecting the interior of the liquid impermeable sleeve (2) to a wound cover (9) so to guide wound exudate into the absorbent core (3) by means of the negative pressure provided by the first connection site (6) comprises a tubular member (7a), a first end of which rests in the innermost layer or on the outer surface of the innermost layer of the stack formed by the at least one fluid distribution layer (4a) and the at least one absorbent layer (5a).

14. An apparatus (11) for negative pressure wound therapy comprising a vacuum pump (8), a wound cover (9) applicable to a wound in a human or animal skin, and an absorbent tube (1, 1', 1", 1"') according to one of claims 1 to 13 connecting the vacuum pump (8) and the wound cover (9).

15. The apparatus (11) according to claim 14, **characterized in that** a pressure sensor (12) is provided in the connection between the second connection site (7) of the absorbent tube (1, 1', 1", 1"') and the wound cover (9), wherein the apparatus (11) further comprises a control unit (13) for controlling the vacuum pump (8), the control unit (13) is arranged to control the vacuum pump (8) by considering the pressure determined by the pressure sensor (12).
